(19) Europäisches Patentamt
European Patent Office
Office européen des brevets

(11) Publication number: 0 070 041
A2

(12) # EUROPEAN PATENT APPLICATION

(21) Application number: 82107279.0

(22) Date of filing: 18.07.80

(51) Int. Cl.³: C 07 C 143/828
C 07 C 161/04, C 07 D 295/22
//C07C143/78

(30) Priority: 20.07.79 US 59153
14.03.80 US 130342
30.05.80 US 152022

(43) Date of publication of application:
19.01.83 Bulletin 83/3

(84) Designated Contracting States:
DE FR GB

(60) Publication number of the earlier application in accordance with Art. 76 EPC: 0 023 141

(71) Applicant: E.I. DU PONT DE NEMOURS AND COMPANY
Legal Department 1007 Market Street
Wilmington Delaware 19898(US)

(72) Inventor: Levitt, George
3218 Romilly Road
Wilmington Delaware 19810(US)

(72) Inventor: Sauers, Richard Frank
504 Revere Court Hickory Hill
Hockessin Delaware 19707(US)

(74) Representative: Hildyard, Edward Martin et al,
Frank B. Dehn & Co. Imperial House 15-19 Kingsway
London WC2B 6UZ(GB)

(54) **Substituted benzenesulfonyl isocyanates and isothiocyanates and processes for their preparation.**

(57) Isocyanates and isothiocyanates of the general formula

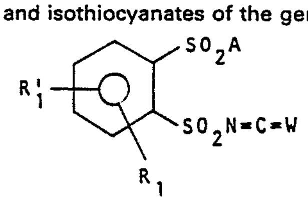

wherein

A is substituted amino, haloalkoxy or optionally substituted phenoxy;

$R_1$ is H, halogen, optionally substituted alkyl, $NO_2$, $OCH_3$, alkylcarbonyl, NCO, $N(CH_3)_2$, CN, $SCH_3$ or $SO_2CH_3$;

$R'_1$ is H, halogen, $CH_3$ or $OCH_3$;

W is O or S;

are valuable intermediates for the preparation of herbicidal sulfonylureas. The novel compounds may be made e.g. by reacting the corresponding sulfonamide with phosgene to obtain the isocyanate, or with carbon disulfide and then with phosgene to obtain the isothiocyanate.

EP 0 070 041 A2

Croydon Printing Company Ltd.

"Substituted benzenesulfonyl isocyanates and isothiocyanates and processes for their preparation".

This invention relates to benzenesulfonyl isocyanates and isothiocyanates having a 2-sulfonate ester or 2-sulfonamido substituent, and to processes for their preparation.

The novel compounds of this invention have the general formula:

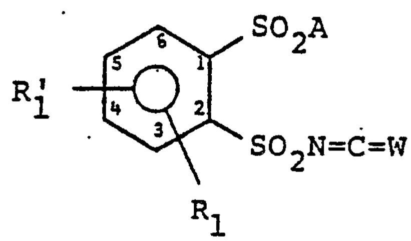

I-B

wherein

A is $NR_2R_3$, $OCH_2CCl_3$, $OCH_2CBr_3$, O 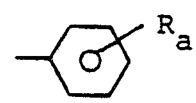

$OCH_2CF_2R_b$ or $\underset{CF_3}{OCHR_c}$ where $R_a$ is H, Cl, $CH_3$, $OCH_3$ or $NO_2$ and $R_b$ is H, F or $C_1$-$C_2$ alkyl with 0-5F and $R_c$ is $CH_3$ or $CF_3$;

$R_1$ is H, Cl, Br, F, $C_1$-$C_3$ alkyl, $NO_2$, $OCH_3$, $\overset{O}{\overset{\|}{C}}R_d$, $CH_2OR_d$, $CF_3$, N=C=O, $N(CH_3)_2$, CN, $S(O)_nCH_3$ or $CH_2S(O)_nCH_3$ where n is 0 or 2 and $R_d$ is $C_1$-$C_3$ alkyl;

$R_1'$ is H, Cl, F, Br, $CH_3$ or $OCH_3$;

W is O or S;

$R_2$ is $C_1$-$C_6$ alkyl, $C_3$-$C_4$ alkenyl, $C_3$-$C_6$ cycloalkyl, $C_5$-$C_6$ cycloalkenyl, $C_4$-$C_7$ cycloalkylalkyl, $C_3$-$C_6$ cycloalkyl substituted with 1-2 $CH_3$ groups, $C_3$-$C_5$ alkynyl, $CF_2CF_2H$, $CF_2CHFCl$, $CF_2CHFBr$, $CF_2CHFCF_3$, $C(CH_3)_2CN$, $(CH_2)_mCN$, where m is 1 or 2, $CH_2CH_2OCH_3$, $CH_2CH(CH_3)OCH_3$, $(CH_2)_3OCH_3$, $CHR_7CO_2R_8$ or $CHR_7CON(R_8)_2$, where $R_7$ is H or $CH_3$ and $R_8$ is $C_1$-$C_3$ alkyl, $OCH_3$,

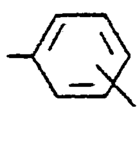 , 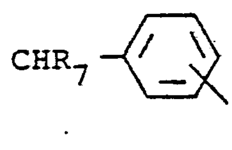 or 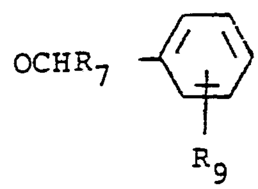

where

$R_9$ is H, $CH_3$, Cl, Br or F;

$R_3$ is $C_1$-$C_4$ alkyl, $C_3$-$C_4$ alkenyl, $CH_2CH_2OCH_3$, $CH_2CH(CH)_3OCH_3$, $CH_2CF_3$ or $(CH_2)_mCN$, where m is 1 or 2 or $CHR_7CO_2R_8$ or $NR_2R_3$ taken together are

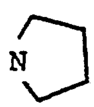 , 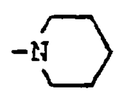 , 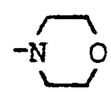 or 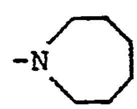

provided that:

1) when $R_2$ is $OCH_3$, $R_3$ is $CH_3$;

2) when $R_2$ is $CF_2CHFCl$, $CF_2CHFBr$, $CF_2CF_2H$ or $CF_2CHFCF_3$, then $R_3$ is $C_1$-$C_4$ alkyl;

3) when $R_1$ is CN or N=C=O, it cannot be in the 3-position.

The compounds of general formula (I) are valuable intermediates for the preparation of the sulfonylurea derivatives disclosed in our copending EP-A-0,023,141, to which the reader is referred for a full disclosure of the utility of these compounds. The said sulfonylurea derivatives have potent herbicidal and plant growth regulant effects. The particular nature and orientation of the substituents $SO_2A$ and $R_1'$ in the compounds of the present invention result in sulfonylurea final products having unexpectedly valuable properties.

Preferred compounds of general formula (I) for reasons of higher activity and/or lower cost of the sulfonylurea herbicides derivable therefrom are:

1) Compounds of Formula (I) in which W = O.

2) Compounds of preference (1) in which $R_1' = H$.

3) Compounds of preference (2) wherein A is $OCH_2CF_3$ or $NR_2R_3$ and $R_2$ is $C_1$-$C_6$ alkyl, $C_3$-$C_4$ alkenyl, $OCH_3$, $CH_2CH_2OCH_3$, $CH_2CH(CH_3)OCH_3$ or $(CH_2)_3OCH_3$, or $NR_2R_3$ taken together are

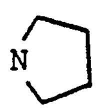 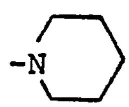 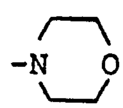 or 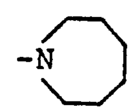.

4) Compounds of preference (3), wherein $R_3$ is $C_1$-$C_4$ alkyl or where $NR_2R_3$ taken together are

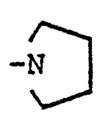 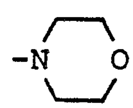 or 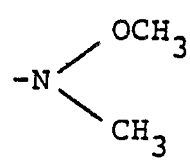.

5) Compounds of preference (4), wherein $R_1$ is H, Cl, $CF_3$, $NO_2$, $CH_3$ or $OCH_3$.

6) Compounds of preference (5), wherein $R_2$ is $C_1$-$C_4$ alkyl or $NR_2R_3$ taken together are

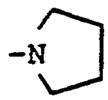 , 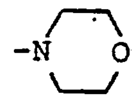 .

Specifically preferred compounds of general formula (I) for reasons of highest activity and/or lowest cost of sulfonylurea herbicides derivable therefrom are:

N,N-Dimethyl-2-(isocyanatosulfonyl)benzenesulfonamide.

N-Ethyl-2-(isocyanatosulfonyl)-N-methylbenzenesulfonamide.

2-(Isocyanatosulfonyl)-N-methyl-N-(methylethyl)benzenesulfonamide.

N,N-Dimethyl-2-(isocyanatosulfonyl)-4-trifluoromethylbenzenesulfonamide.

2-[(2,2,2-Trifluoroethyl)sulfonyl]benzenesulfonyl isocyanate.

## Synthesis

The synthesis of our compounds is described in Equations 1-3.

### Equation 1

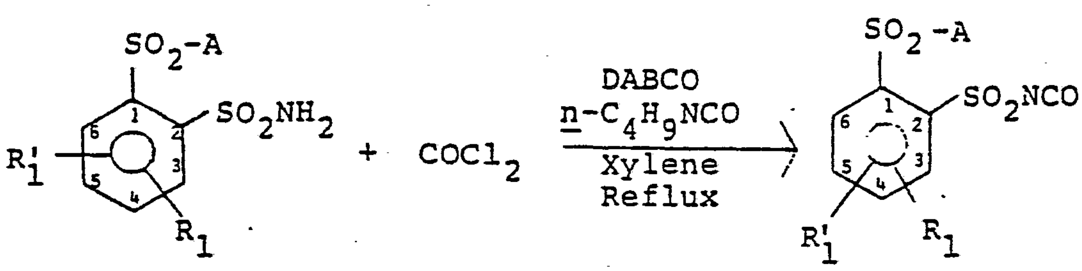


II    Ia

wherein

A is $NR_2R_3$, $OCH_2CCl_3$, $OCH_2CBr_3$, 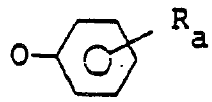, $OCH_2CF_2R_b$ or $\underset{CF_3}{OCHR_c}$ where $R_a$ is H, Cl, $CH_3$, $OCH_3$ or $NO_2$ and $R_b$ is H, F or $C_1$-$C_2$ alkyl with 0-5F and $R_c$ is $CH_3$ or $CF_3$.

$R_1$ is H, Cl, Br, F, $NO_2$, $C_1$-$C_3$ alkyl, $OCH_3$, $CF_3$, $\overset{O}{\overset{\|}{C}}R_d$ or $CH_2OR_d$ where $R_d$ is $C_1$-$C_3$ alkyl, $N(CH_3)_2$, CN, $CH_3\overset{(O)_n}{S}-CH_2$ or $CH_3\overset{(O)_n}{S}-$, where n is 0 or 2;

$R_1'$ is H, Cl, F, Br, $CH_3$ or $OCH_3$;

$R_2$ is $C_1$-$C_6$ alkyl, $C_3$-$C_4$ alkenyl, $C_3$-$C_6$ cycloalkyl, $C_5$-$C_6$ cycloalkenyl, $C_4$-$C_7$ cycloalkylalkyl, $C_3$-$C_6$ cycloalkyl substituted with 1-2 $CH_3$ groups, $C_3$-$C_5$ alkynyl, $C(CH_3)_2CN$, $(CH_2)_mCN$, where m is 1 or 2, $CF_2CF_2H$, $CF_2CHFCl$, $CF_2CHFBr$, $CF_2CHFCF_3$, $CH_2CF_3$, $CH_2CH_2OCH_3$, $CH_2CH(CH_3)OCH_3$, $(CH_2)_3OCH_3$, or $OCH_3$, provided that when $R_2$ is $OCH_3$, $R_3$ is $CH_3$, $CH(R_7)CO_2R_8$ or $CH(R_7)CON(R_8)_2$, where $R_7$ is H or $CH_3$, and $R_8$ is $C_1$-$C_3$ alkyl; or $R_2$ is

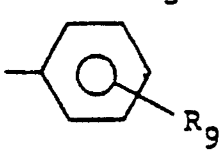 , $CH(R_7)$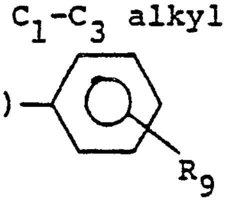 or $OCHF_2$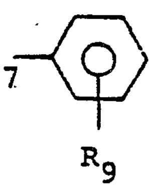

where

$R_9$ is H, $CH_3$, Cl, Br or F;

$R_3$ is $C_1$-$C_4$ alkyl, $C_3$-$C_4$ alkenyl, $CH_2CH_2OCH_3$, $CH_2CH(CH_3)OCH_3$, $(CH_2)_mCN$, where m is 1 or 2, $CH(R_7)CO_2R_8$, $-CH_2CF_3$, or $NR_2R_3$ taken together are

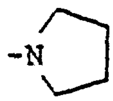 , 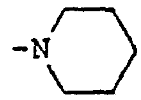 , 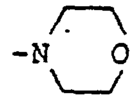 or 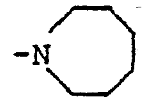 ,

provided that when $R_2$ is $CF_2CHFCl$, $CF_2CHFBr$, $CF_2CF_2H$, or $CF_2CHFCF_3$, then $R_3$ is $C_{1-4}$ alkyl; Also provided that when $R_1$ is CN or N=C=O, it cannot be in the 3-position, and that when $R_2$ is $OCH_3$ $R_3$ is $CH_3$.

The above reaction is carried out by heating a mixture of the appropriate sulfonamide (II), an alkyl isocyanate such as butyl isocyanate and a catalytic amount of a tertiary amine such as 1,4-diaza[2,2,2]bicyclooctane (DABCO) in xylene, or other inert solvent of boiling point $\geq 135°$ to approximately 135°. Phosgene is then added to the mixture over a 1-6 hour period until an excess of phosgene is present as indicated by a drop in the boiling point to less than 130°. The mixture is cooled and filtered to remove a small amount of insoluble by-products. The solvent and the alkyl isocyanate are distilled off in-vacuo leaving a residue of the crude, sulfonyl isocyanate, (Ia), which can be used without further purification.

The preparation of the sulfonamides, II, is described in Equation 4.

The sulfonyl isocyanates, Ia, can also be prepared, as shown in Equation 2, by first preparing an N-alkyl-N'-sulfonylurea, e.g. the n-butylsulfonylurea, III; and reacting the compound III with phosgene and a catalytic amount of a tertiary amine in an inert solvent.

Equation 2

II

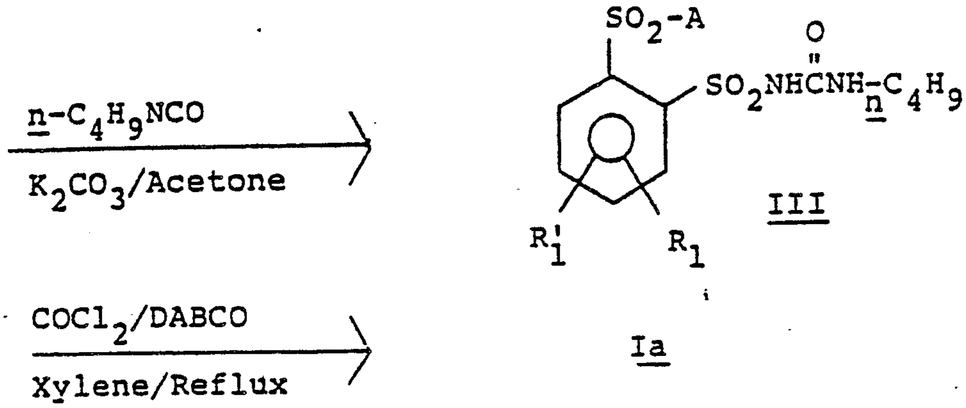


III

III

Ia

wherein

A, $R_1$, $R_1'$, $R_2$ and $R_3$ are as defined in Equation 1.

The compounds III are conveniently prepared by stirring a mixture of the sulfonamides, II, anhydrous potassium carbonate, and n-butyl isocyanate in acetone or methyl ethyl ketone at 25-80° until all of the isocyanate has reacted. The products are isolated by quenching in dilute mineral acid or by distilling off the solvent and recrystallizing the residue. The compounds III are treated with phosgene and a catalytic amount of DABCO in refluxing xylene or chlorobenzene in a manner analogous to that described in Equation 1.

Where W = S in Formula I, the useful isothiocyanate intermediates, IV, (within the scope of general formula I) are prepared according to Equation 3, by reacting the sulfonamide (II) with carbon disulfide and base to form a salt of the corresponding dithiocarbamic acid and reacting said salt with phosgene in the presence of an inert solvent.

Equation 3

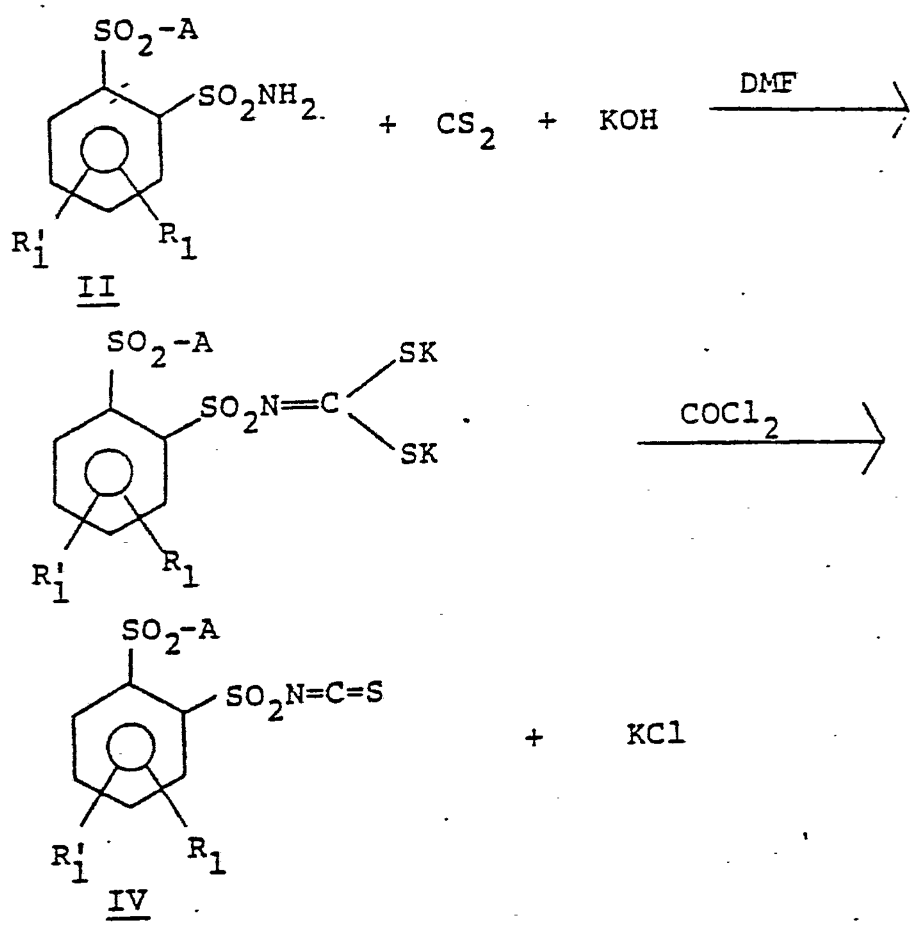


II

IV

wherein

A, $R_1$, $R_1'$, $R_2$ and $R_3$ are as defined in Equation 1.

The substituted sulfonamide is e.g. dissolved in dimethylformamide (DMF) with an equivalent amount of carbon disulfide, and two equivalents of potassium hydroxide are added in portions at room temperature. The mixture is stirred for 1-8 hours then diluted with ethyl acetate, ethyl ether, or other similar aprotic solvent to precipitate the dipotassium salt of the dithiocarbamic acid. The salt is isolated and suspended in an inert solvent such as xylene, chloroform, or carbon tetrachloride. Phosgene is added below room temperature and the mixture stirred at room temperature for 1-3 hours. The sulfonylisothiocyanate

is isolated by filtering off the precipitated potassium chloride and concentrating the filtrate. These compounds tend to dimerize on standing and therefore should be used soon after preparation.

The benzenesulfonamides of Formula II, which are the starting materials in Equations 1, 2 and 3, can be prepared by the four step procedure described in Equation 4.

Equation 4.

(4a)

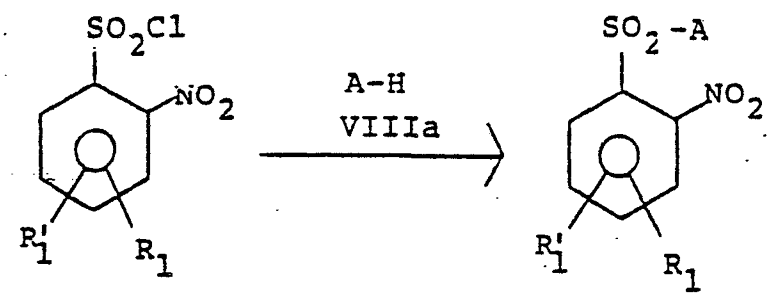


VIII IX

(4b) IX

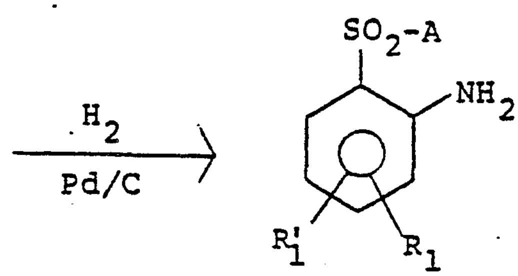


X

(4c) X

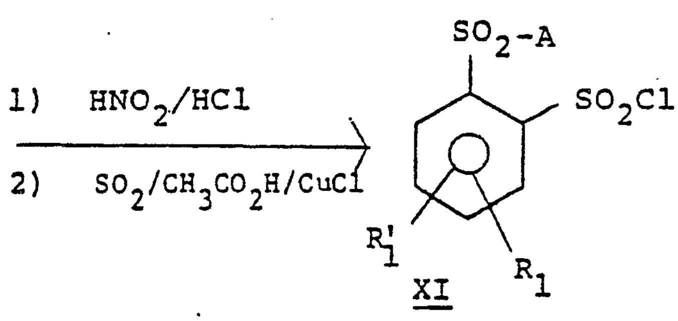


XI

(4d) 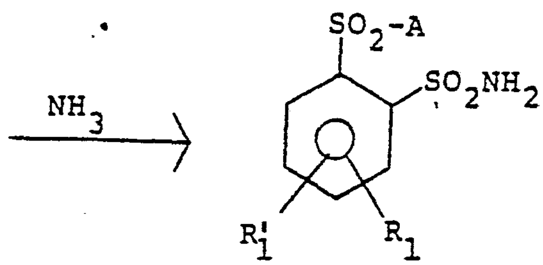

II

wherein

A, $R_1'$ $R_1$, $R_2$, and $R_3$ are as defined in Equations 1-3, with the exception that $R_1$ cannot be $NO_2$.

In step 4a, the o-nitrobenzenesulfonyl chloride in Formula VIII, which are well-known in the art, are treated with an amine or an alcohol of Formula VIIIa in an inert organic solvent such as methylene chloride, ethyl ether, or tetrahydrofuran at 0-50°. When VIIIa is an amine, it may be taken in excess to act as an acid acceptor; or, alternatively, a tertiary amine such as triethylamine or pyrimidine may be used as an acid acceptor. When VIIIa is an alcohol, a tertiary amine such as triethylamine or pyridine must be used as an acid acceptor. The by-product amine hydrochloride is filtered off or washed out of the solvent with water and the product isolated by evaporation of the solvent.

The reduction described in step 4b is accomplished by treating a solution of the compounds of Formula IX, in a solvent such as ethanol, ethyl acetate, or DMF, in a pressure vessel, with 100-1000 pounds per square inch of hydrogen at 80-150° in the presence of a hydrogenation catalyst such as 5-10% palladium absorbed on carbon. When the theoretical amount of hydrogen has been absorbed, the solution is cooled and the catalyst is removed by filtration. The product is then isolated by evaporation of the solvent.

In the case where $R_1 = NO_2$, the reduction of step 4b can be accomplished using ammonium sulfide or sodium hydrosulfide instead of catalytic hydrogenation. This type of procedure is described in Organic Synthesis Coll. Vol. III, pgs. 242-3, John Wiley and Sons, Inc., New York and London (1955), to which the reader is referred for further information.

The diazotization and coupling with sulfur dioxide, described in step 4c, is accomplished in the following manner. A solution of the aniline of Formula X in a mixture of concentrated hydrochloric acid and glacial acetic acid is treated with a solution of sodium nitrite in water at -5 to 0°. After stirring for 10-15 minutes at 0° to insure complete diazotization, this solution is added to a mixture of an excess of sulfur dioxide, and a catalytic amount of cuprous chloride in glacial acetic acid at 0-5°. The temperature is kept at 0-5° for 1/4 to 1 hour then raised to 20-25° and held at that temperature for 2-4 hours. This solution is then poured into a large excess of ice water. The sulfonyl chloride products, XI, can be isolated by filtration or by extraction into a solvent such as ethyl ether or methylene chloride followed by evaporation of the solvent.

The amination described in step 4d is conveniently carried out by treating a solution of the sulfonyl chloride of Formula XI with an excess of anhydrous ammonia in a solvent such as ethyl ether or methylene chloride at 0-25°. If the product sulfonamide, II, is insoluble it may be isolated by filtration followed by washing out the salts with water. If the product sulfonamide is soluble in the reaction solution, it may be isolated by filtering off the precipitated ammonium chloride and evaporation of the solvent.

Compounds of Formula IX, which are intermediates in the preparation of the compounds of Formula II - as described in Equation 4, can also be prepared by the procedures shown in Equations 5 and 6.

Equation 5

5a) 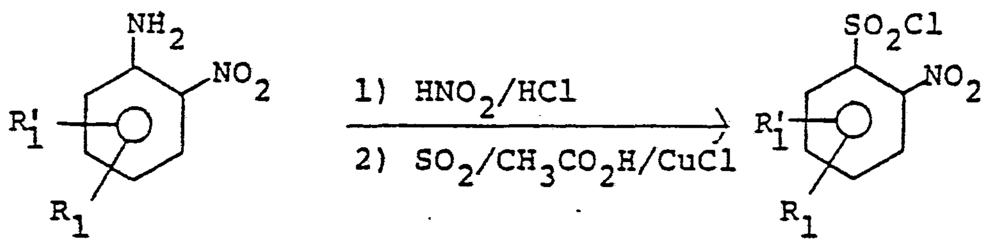


XVII VIII

5b) 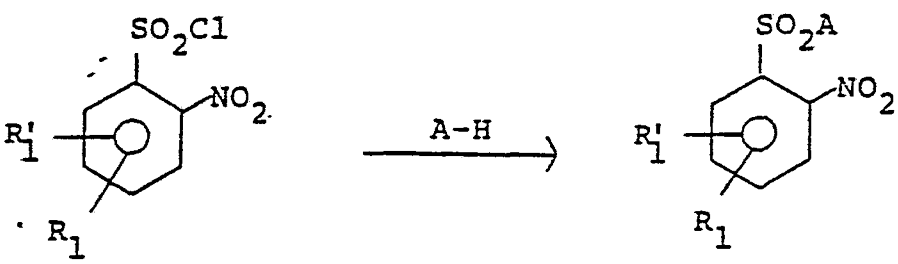


VIII IX

wherein

A, $R_1$ and $R_1'$ are as described in Equation 1.

The o-nitroanilines of Formula XVII are well known in the art. The method of converting compounds XVII to compounds VIII is analogous to that described in Equation (4c). The procedures for converting compounds VIII to compounds IX are described in Equation (4.a).

Equation 6

6a) 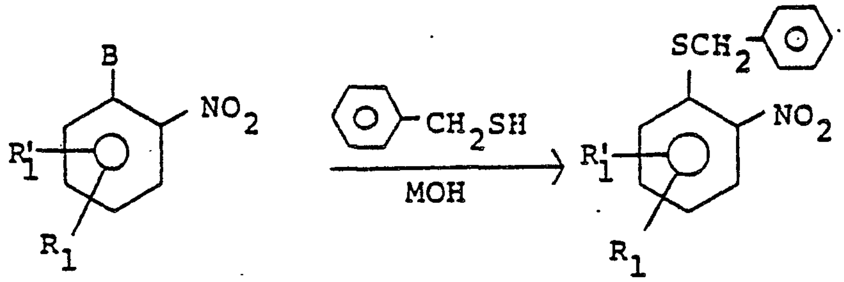


XVIII XIX

6b) 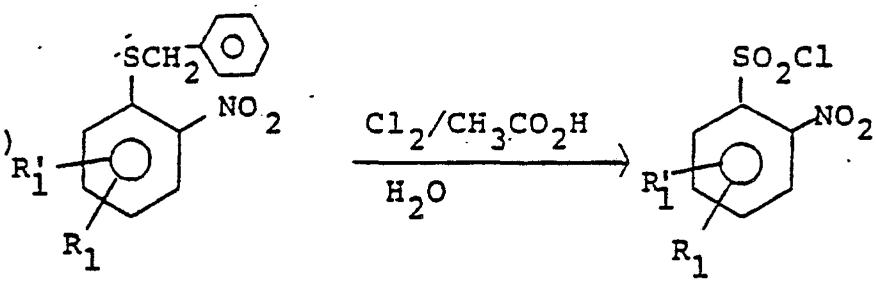


XIX VIII

6c) 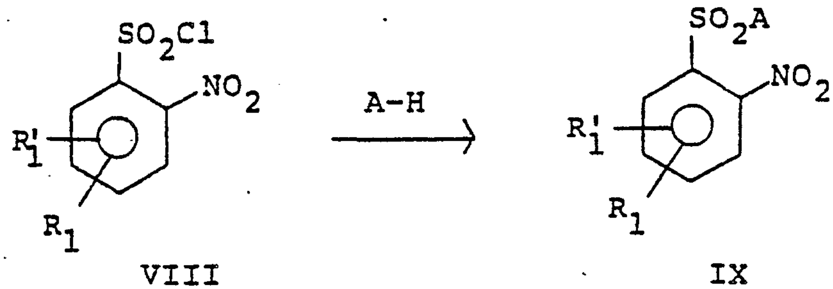


VIII IX

wherein

A, $R_1$, and $R_1'$ are as described in Equation 1;

B is Cl or Br; and

M is Na or K.

In Step (6a), the compounds of Formula XVIII, which are well known in the art, are reacted with benzyl mercaptan in the presence of an equivalent amount of a base such as sodium or potassium hydroxide. The preferred solvent for this reaction is an alcohol

such as methyl, ethyl or isopropyl alcohol. The reaction can be carried out at temperatures between 25 and 80° but temperatures of 50-80°C are preferred. The products of Formula XIX are isolated by cooling the reaction mixture to 0-20°, filtering off the precipitated product, and washing this solid with water to remove the by-product alkali halide.

In Step (6b), the compound of Formula XIX is suspended in a mixture of 85-95% acetic acid and 5-15% water. This suspension is treated with at least three molar equivalents of chlorine at 0-20°. When a clear solution is obtained, the reaction solution is quenched in an excess of ice water and extracted with a solvent such as methylene chloride, chloroform or 1-chlorobutane. The products of Formula VIII are isolated by concentrating this solvent extract <u>in vacuo</u> to remove the solvent and the by-product benzyl chloride. The products are oils or low melting solids and may be used directly in the next step or recrystallized first from such solvents as cyclohexane or 1-chlorobutane.

Compounds of Formula IX, in which A includes certain fluoroalkylamine moieties can also be prepared by the procedure shown in Equation 7.

Equation 7

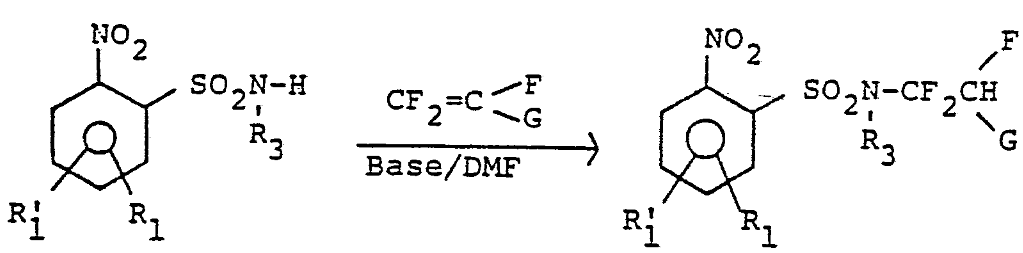


IX IXa

wherein

$R_1$ and $R_1'$ are as previously described; $R_3$ is $C_1$-$C_4$ alkyl, and G is F, Cl, Br or $CF_3$.

The reaction described in Equation 7 is accomplished by heating a mixture of the sulfonamide IX, one or two equivalents of the fluoroolefin and a catalytic amount of base in dimethylformamide solution in a pressure vessel at a temperature of between 60 and 130° until a pressure drop is no longer observed. Appropriate bases for this reaction include potassium hydroxide, sodium hydroxide, sodium hydride and sodium metal. The products of the reaction are isolated by quenching the reaction mixture in a large excess of water and extraction with *n*-butyl chloride or diethylether. Evaporation of the solvent yields the desired sulfonamides IXa as viscous oils.

Compounds of Formula IX in which A includes a benzyloxyamine group may also be prepared by the procedure described in Equation 8.

Equation 8

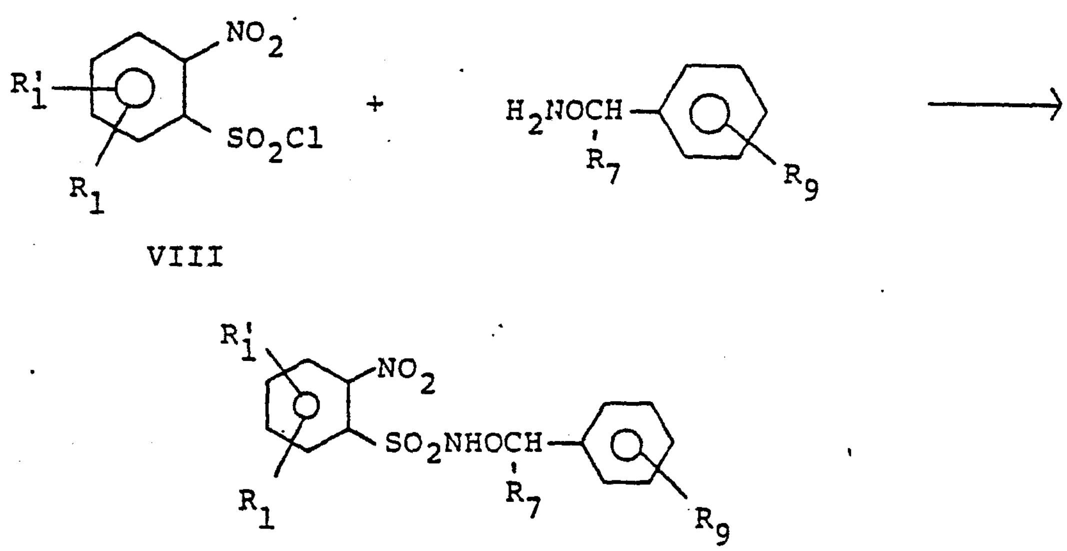

VIII

IXc

IX

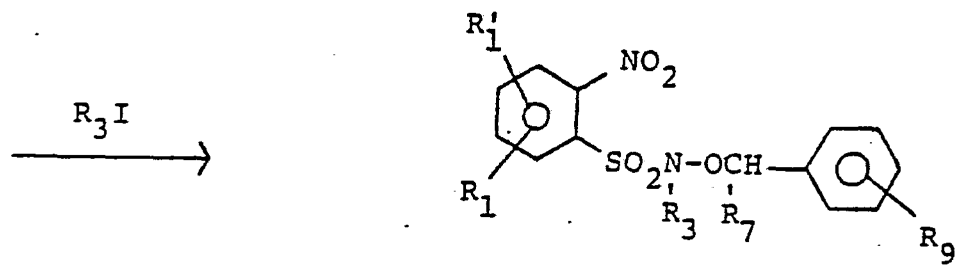


IXd

wherein

$R_1$, $R_1'$, $R_3$, $R_7$ and $R_9$ are as previously described.

In the first step of Equation 8, a sulfonyl chloride of Formula XIII is reacted with an appropriate benzyloxyamine, taken in excess to act as an acid acceptor, in diethyl ether or tetrahydrofuran solution at 0-10°. The reaction is completed by stirring at ambient temperature for 4-24 hours. The solvent is evaporated and the product sulfonamide IXc purified by washing the residue with water to remove the by-product amine hydrochloride.

In the second step of Equation 8, the sulfonamide of Formula IXc is stirred at ambient temperature for 18-24 hours with an excess of both an alkyl iodide and anhydrous potassium carbonate or sodium carbonate in acetone or 2-butanone solution. The solvent is evaporated and the residue partitioned between methylene chloride and water. Concentration of the methylene chloride solution affords the compounds of Formula IXd as pale yellow oils.

The compounds of Formula IXd may be converted to compounds of Formula Xd by the procedure described in Equation 9.

Equation 9

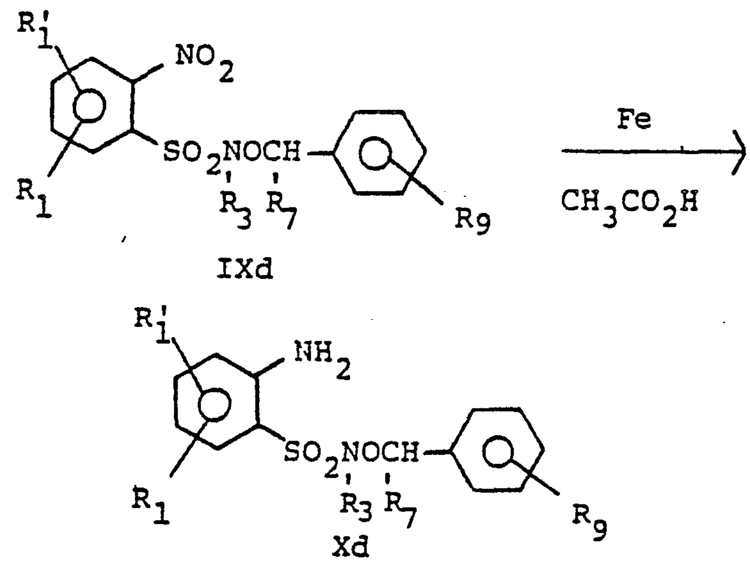


IXd

Xd

wherein

$R_1$, $R_1'$, $R_3$, $R_7$ and $R_9$ are as previously described.

The conversion in Equation 9c is accomplished in the following manner: A solution of the nitro compound IXd in 90% aqueous acetic acid is treated with an excess of iron powder (5 g - atom/mole of IXd) at 80-90° in several portions. After heating an additional 20 minutes, charcoal is added and the solution filtered through Celite® and concentrated *in-vacuo*. The residue is made strongly alkaline with 20% sodium hydroxide then extracted with methylene chloride. Concentration of the organic solution yields the anilines of Formula Xd as viscous oils.

The compounds of Formual Xd may be converted to the compounds of Formula I by the procedures described in Equations 1-3.

The conversion of compounds VIII to compounds IX is described in Equation (4a).

Compounds of Formula I in which $R_1$ is -N=C=O, can be prepared by the procedures described in Equation 10.

Equation 10

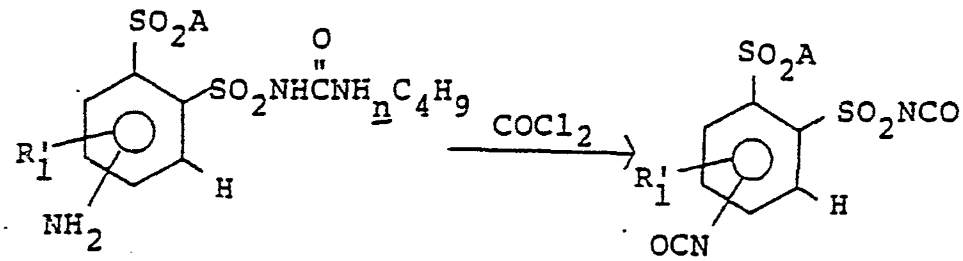


IIIa Ib

wherein

A, $R_1'$, $R_5$ and $R_6$ are as previously described.

In Equation 10 the compounds of Formula IIIa, which are well known in the art, are added to at least two equivalents of phosgene in an inert solvent such as xylene or chlorobenzene at 0-5°. A slow stream of phosgene is then introduced and the reaction temperature raised to 130° (reflux). Refluxing the reaction mixture for 1-2 hours, followed by purgation with nitrogen, filtration, and concentration yields the products of Formula Ib, as viscous oils. These compounds may be further purified by vacuum distillation.

The following Examples illustrate the use of reaction sequences described above to prepare compounds of general formula (I). Temperatures are given in degrees centigrade.

Example 1

o-Nitro-N,N-diethylbenzenesulfonamide

To a solution of 132.6 g of o-nitrobenzenesulfonyl chloride in 700 ml of tetrahydrofuran was added 88.5 g of diethylamine at 5-15°. The reaction mixture was stirred at room temperature for 1 hour before the precipitated diethylamine hydrochloride was removed by filtration. The filtrate was evaporated to dryness in-vacuo and the residue dissolved in 1-chlorobutane. The 1-chlorobutane solution was washed with water, dried over magnesium sulfate and evaporated in-vacuo to give 122.4 g of o-nitro-N,N-diethylbenzenesulfonamide as a dark oil.

NMR($CDCl_3$)$\delta$: 1.1-1.4 [t, 6.1H, $(CH_3CH_2)_2N-$];
3.3-3.8 [qt, 3.8H, $(CH_3CH_2)_2N-$];
8.0-8.6 (m, 4.1H, 4 aromatics).

Example 2

o-Amino-N,N-diethylbenzenesulfonamide

In a pressure vessel a mixture of 133 g of o-nitro-N,N-diethylbenzenesulfonamide, 5 g of 10% palladium on carbon, and 500 ml of ethyl acetate was shaken at 130° under 500 psi hydrogen pressure until hydrogen was no longer absorbed. The reaction mixture was cooled and the catalyst filtered off. Evaporation of the solvent in-vacuo gave 123 g of o-amino-N,N-diethylbenzenesulfonamide as a viscous oil which slowly crystallized to a solid, m.p. 45-51°.

NMR($CDCl_3$)$\delta$: 1.0-1.3 [t, 6.7H, $(CH_3CH_2)_2N-$];
3.0-3.5 [qt, 3.6H, $(CH_3CH_2)_2N-$];
4.8-5.2 (broad, 1.7H, $NH_2$);
6.5-7.7 (m, 4.0H, 4 aromatics).

Example 3

N,N-Diethyl-1,2-benzenedisulfonamide

To a solution of 114 g of o-amino-N,N-diethylbenzenesulfonamide in a mixture of 400 ml of concentrated hydrochloric acid and 100 ml of glacial acetic acid was added a solution of 50 g of sodium nitrite in 130 ml of water at -5 to 0°. The solution was stirred at 0° for 15 minutes then poured into a mixture of 14 g of cuprous chloride and 100 ml of liquid sulfur dioxide in 550 ml of glacial acetic acid at 0-5°. This mixture was stirred at 0° for 15 minutes then at room temperature for 3 hours before pouring into three liters of ice water. The crude sulfonyl chloride was filtered off and washed with water. It was then dissolved in 1 ℓ of ethyl ether, washed with water and dried over magnesium sulfate. To this ether solution was added 20 ml of liquid anhydrous ammonia at 5-15°. After stirring overnight at room temperature the solid was filtered off, washed with water, ethanol and then 1-chlorobutane, Oven drying at 60° gave 91.8 g N,N-diethyl-1,2-benzenedisulfonamide, m.p. 156-9°.

NMR(DMSO)δ: 0.9-1.2 [t, 6.0H, $(CH_3CH_2)_2N-$];
3.2-3.6 [qt, 3.8H, $(CH_3CH_2)_2N-$];
∿7.2 (broad singlet, 2.1H, $NH_2$);
7.7-8.4 (m, 4.1H, 4 aromatics).

Example 4

o-N,N-Diethylsulfamoylbenzenesulfonyl isocyanate

A solution of 13.2 g of N,N-diethyl-1,2-benzenedisulfonamide, 4.5 g of n-butylisocyanate, and 0.2 g of 1,4-diaza[2,2,2]bicyclooctane (DABCO) in 90 ml of mixed xylenes was heated to 135°. To this solution was added 3.3 ml of liquid phosgene at such a rate that the temperature was maintained between 125 and 135° (about 2 hours). The temperature was kept at 130° for 1/2 hour after the addition. The solution

was cooled and filtered to remove a small amount of insoluble solid then concentrated at 60-70° in-vacuo. The residue of o-N,N-diethylsulfamoylbenzenesulfonyl isocyanate was an oil weighing 16.8 g and was sufficiently pure for further reaction.

Example 5

2-Nitro-1-[(phenylmethyl)thio]-4-trifluoromethylbenzene

To a mixture of 225.6 g of 4-chloro-3-nitrobenzotrifluoride and 136 g of benzyl mercaptan in 1.5 ℓ of ethyl alcohol, at reflux, was added a solution of 72.5 g of 85% KOH in 120 ml of water over a 45 minute period. Reflux was continued for an additional three hours. The reaction mixture was cooled to 5°C. The precipitate was filtered off, washed with ethanol and water and dried to give 273.2 g of 2-nitro-1-[(phenylmethyl)thio]-4-trifluoromethylbenzene as a bright yellow solid, m.p. 132-136°.

NMR(DMSO-$d_6$)δ: 4.45 (s, 1.9H, $SCH_2Ø$);
7.2-7.7 (m, 5.0H, 5 aromatics);
7.9-8.2 (m, 2.1H, 2 aromatics);
8.5 (s, 1.0H, 1 aromatic).

Example 6

N,N-Dimethyl-2-nitro-4-(trifluoromethyl)benzenesulfonamide

To a suspension of 156.5 g of 2-nitro-1-[(phenylmethyl)thio]-4-trifluoromethylbenzene in a mixture of 800 ml of acetic acid and 100 ml of water was added 78 ml of liquid chlorine at 10-18° over a 1 hour period as the suspension gradually turned into a clear solution. The solution was stirred at 18-25° for 2 hours, then poured into 1 ℓ of ice water. The mixture was extracted with 1-chlorobutane and the

1-chlorobutane extract washed well with water. The solution was dried over $MgSO_4$ and stripped, finally at 70-80°/1-2 mm, to give 164 g of crude sulfonyl chloride as a light yellow oil. This oil was dissolved in 600 ml of methylene chloride, cooled to 5°, and treated with 75 ml of liquid dimethylamine at 5-15°. After stirring at 25° for 1 hour, the reaction mixture was washed with water, dried over $MgSO_4$, and stripped *in vacuo* to give a crude solid. This solid was slurried in ethanol, filtered and washed with ethanol, and dried to give 109.1 g of N,N-dimethyl-2-nitro-4-(trifluoromethyl)benzenesulfonamide, m.p. 102-104.5°.

NMR($DMSO-d_6$)δ: 2.9 (s, 6.1H, $NMe_2$);
8.3 (s, 2.0H, 2 aromatics);
8.7 (s, 0.9H, 1 aromatic).

Anal. Calcd. for $C_9H_9F_3N_2O_4S$: C, 36.25; H, 3.04;
N, 9.39; S, 10.75.
Found: C, 36.4, 3.65; H, 3.00, 3.04;
N, 9.58, 9.52; S, 11.1, 11.0.

Example 7

2-Amino-N,N-dimethyl-4-(trifluoromethyl)benzenesulfonamide

In a pressure vessel, 109 g of N,N-dimethyl-2-nitro-4-(trifluoromethyl)benzenesulfonamide, 250 ml of ethyl acetate and 5 g of 10% palladium-on-carbon were shaken at 130° under 500 psi hydrogen pressure until no more hydrogen was absorbed, as determined by no additional pressure drop. The reaction mixture was cooled and the catalyst filtered off. The solution was stripped *in vacuo* to give 95.2 g of 2-amino-N,N-dimethyl-4-(trifluoromethyl)benzenesulfonamide as a slowly crystalizing oil, m.p. 65-70°C.

NMR($CDCl_3$)δ: 2.8 (s, 5.8H, $NMe_2$);
5.1-5.6 (broad, 2.2H, $NH_2$);
6.9-7.9 (m, 3.0H, 3 aromatics).

Example 8

$N^1$,$N^1$-Dimethyl-4-(trifluoromethyl)-1,2-benzenedisulfonamide

To a suspension of 38.0 g, of 2-amino-N,N-dimethyl-4-(trifluoromethyl)benzenesulfonamide in a mixture of 115 ml of concentrated hydrochloric acid and 40 ml of glacial acetic acid was added a solution of 14.0 g of sodium nitrite in 40 ml of water at -5 to 0° over a 30 minute period. The resulting diazonium solution was stirred at 0° for 15 minutes. This diazonium solution was then added to a solution of 4.0 g of cuprous chloride and 26 ml of liquid sulfur dioxide in 175 ml of glacial acetic acid at 5° over a 10 minute period. The resulting mixture was stirred at 0° for 1 hour and at 25° for 2 hours before pouring into 1 ℓ of ice water. The precipitated sulfonyl chloride which formed was filtered off and washed well with water. The still-wet sulfonyl chloride was suspended in 350 ml of diethyl ether, cooled to 5° and treated with 7.0 ml of liquid ammonia. The reaction mixture was stirred at room temperature for 1 hour. The precipitate was filtered-off, washed with ether, water and then ethanol to give 22.4 g of $N^1$,$N^1$-dimethyl-4-(trifluoromethyl)-1,2-benzenedisulfonamide as a white solid, m.p. 189-191°.

NMR(DMSO-$d_6$)δ: 2.7 (s, 6.1H, $-NMe_2$);
7.3 (broad singlet, 1.9H, $-SO_2NH_2$);
8.0-8.4 (m, 3.0H, 3 aromatics).

Example 9

N'-(Butylaminocarbonyl)-N,N-dimethyl-4-(trifluoromethyl)-1,2-benzenedisulfonamide

A mixture of 49.7 g of N',N'-dimethyl-4-(trifluoromethyl)-1,2-benzenedisulfonamide 22.5 g of *n*-butylisocyanate, and 31.1 g of anhydrous potassium carbonate in 600 ml of 2-butanone was stirred at reflux for four hours. The mixture was cooled to 25° and poured into 1.6 ℓ of ice water. The solution was acidified to pH = 1 with concentrated hydrochloric acid. The precipitate was filtered off and washed with water and 1-chlorobutane. Drying overnight at 60° *in vacuo* gave 55.6 g of N'-(butylaminocarbonyl)-N,N-dimethyl-4-(trifluoromethyl)-1,2-benzenedisulfonamide as a white solid, m.p. 128-130°.

NMR(DMSO-$d_6$)δ: 0.8-1.6 (m, 7.7H, 7 butyl hydrogens);
2.8-3.5 (m, 7.5H, $NMe_2$ + $C\underline{H}_2NH$);
6.8-7.1 (m, 0.9H, $N\underline{H}CH_2$);
8.4-8.8 (m, 3.1H, 3 aromatics);
∿10.3 (broad, 0.9H, NH).

Example 10

2-(N,N-Dimethylsulfamoyl)-5-(trifluoromethyl)benzenesulfonylisocyanate

A solution of 12.5 g of N'-(butylaminocarbonyl)-N,N-dimethyl-4-(trifluoromethyl)-1,2-benzenedisulfonamide, and 0.1 g of DABCO in 75 ml of mixed xylenes was heated to 136°. To this solution was added 2.2 ml of liquid phosgene over a period of two hours at a rate that the temperature was maintained between 125° and 135°. The temperature was kept at 130° for 1/2 hour after the addition. The solution was cooled and filtered under a nitrogen atmosphere to remove a small amount of insoluble solid then concentrated

at 60-70° *in vacuo*. The residue of 2-(N,N-dimethylsulfamoyl)-5-(trifluoromethyl)benzenesulfonylisocyanate was an oil weighing 10.4 g and was sufficiently pure for further reaction.

Example 11

2,2,2-Trifluoroethyl 2-nitrobenzenesulfonate

To a solution of 110.5 g of *o*-nitrobenzenesulfonyl chloride and 50.0 g of 2,2,2-trifluoroethanol in 600 ml of diethyl ether was added 52.0 g of triethylamine at 5-15° over a 45 minute period. The reaction mixture was stirred at 25° for 1 hour then washed with water, dried over $MgSO_4$, and stripped *in vacuo* to give 2,2,2-trifluoroethyl 2-nitrobenzenesulfonate as an oil weighing 110.3 g.

NMR($CDCl_3$)δ: 4.3-4.8(qt. 1.8H, $CH_2CF_3$)
7.8-8.4(m, 4.2H, 4 aromatics).

Example 12

2,2,2-Trifluoroethyl 2-aminobenzenesulfonate

In a pressure vessel, 107 g of 2,2,2-trifluoroethyl 2-nitrobenzenesulfonate and 8 g of 5% palladium-on-carbon in 300 ml of ethyl acetate was heated to 110° and shaken under 500 psi hydrogen pressure until hydrogen was no longer absorbed. The reaction mixture was cooled and the catalyst filtered off. The filtrate was stripped *in vacuo* to give 87.5 g of 2,2,2-trifluoroethyl 2-aminobenzenesulfonate as a dark oil.

NMR($CDCl_3$)δ: 4.0-4.4(qt. 2.0H, $CH_2CF_3$);
4.8-5.4(broad, 1.9H, $NH_2$);
6.5-7.8(m, 4.1H, 4 aromatics).

Example 13

2,2,2-Trifluoro 2-(aminosulfonyl)benzenesulfonate

Using a procedure analogous to that described in Example 10, 63.8 g of the product of Example 12 was converted to 38.4 g of 2,2,2-trifluoro 2-(aminosulfonyl)benzenesulfonate, m.p. 97-100° (recrystallized from 1-chlorobutane).

NMR(DMSO-$d_6$)δ: 4.4-4.9(qt. 1.9H, $CH_2CF_3$); 6.8-7.1(broad singlet, 1.9H, $SO_2NH_2$); 7.7-8.6(m, 4.2H, 4 aromatics).

Example 14

(2,2,2-Trifluoroethyl) 2-[[(butylamino)carbonyl]aminosulfonyl]benzenesulfonate

Using a procedure analogous to that described in Example 9, 35.1 g of the product of Example 13 was converted to 45.0 g of (2,2,2-trifluoroethyl) 2-[[butylamino)carbonyl]aminosulfonyl]benzenesulfonate, m.p. 140-2.5°.

NMR(DMSO-$d_6$)δ: 0.7-1.4(m, 7.2H, 7 butyl hydrogens); 2.7-3.2(m, 2.1H, 2 butyl hydrogens); 4.6-5.1(qt, 2.0H, $CH_2CF_3$); 6.5-6.9(broad t, 0.9H, $NHCH_2$); 7.9-8.6(m, 3.8H, 4 atomatics).

Example 15

2,2,2-Trifluoroethyl 2-(isocyanatosulfonyl)benzenesulfonate

Using a procedure analogous to that described in Example 10, 20.6 g of the product of Example 14 was converted to 16.6 g of 2,2,2-trifluoroethyl 2-(isocyanatosulfonyl)benzenesulfonate, obtained as a crude oil.

## Example 16

N,N-Dimethyl-4-methoxy-2-nitrobenzenesulfonamide

A mixture of 84.0 g of 4-methoxy-2-nitroaniline, 400 ml of conc. hydrochloric acid, and 200 ml of acetic acid was treated with a solution of 50 g of sodium nitrite in 120 ml of water over a 45 minute period at -5 to 0°. The resulting solution was stirred at 0° for 15 minutes. This diazonium solution was added to a solution of 12 g of cuprous chloride and 100 ml of sulfur dioxide in 600 ml of acetic acid over 15 minutes at 0-5°. The mixture was stirred at 0° for 1 hour and at 25° for 2 hours before pouring into 4 ℓ of ice water. The precipitated sulfonyl chloride was filtered and washed well with water. The still-wet sulfonyl chloride was suspended in 600 ml of diethyl ether, cooled to 0°, and treated with 55 ml of liquid dimethyl amine at 0-10°. After stirring at room temperature for 30 minutes, the precipitate was filtered and washed with ether, then water. Drying at 60° in vacuo gave 92.0 g of N,N-dimethyl-4-methoxy-2-nitrobenzenesulfonamide as a white solid, m.p. 90-92°.

NMR($DMSO-d_6$)δ: 2.7(s, 6.1H, $-NMe_2$);
3.9(s, 2.9H, $OCH_3$);
7.2-8.0(m, 2.9H, 3 aromatics).

## Example 17

N,N-Dimethyl-2-amino-4-methoxybenzene-sulfonamide

In a procedure analogous to that described in Example 7, 90.0 g of the product of Example 16 was converted to 66.4 g of N,N-dimethyl-2-amino-4-methoxybenzenesulfonamide, obtained as a dark, slowly crystalizing oil, m.p. 59-64°.

NMR ($CDCl_3$) δ: 2.7 (s, 5.7H, $NMe_2$); 3.7 (s, 3.0H, $OCH_3$); 4.9-5.3 (broad, 2.0H, $NH_2$); 6.2-6.5 (m, 2.2H, 2 aromatics); 7.3-7.6 (m, 1.2H, 1 aromatic).

Example 18

$N^1$, $N^1$-Dimethyl-4-methoxybenzene-1,2-disulfonamide

Using a procedure analogous to that described in Example 8, 58.0 g of the product of Example 17 was converted to 34.8 g of $N^1$, $N^1$-dimethyl-4-methoxybenzene-1,2-disulfonamide, m.p. 106-9°.

NMR (DMSO-$d_6$) δ: 2.8 (s, 5.9H, $-NMe_2$); 3.9 (s, 3.1H, $OCH_3$); 7.0-8.1 (m, 5.0H, 5 aromatics + $SO_2NH_2$).

Example 19

N'-(Butylaminocarbonyl)-N,N-dimethyl-4-methoxy)-1,2-benzenedisulfonamide

Using a procedure analogous to that described in Example 9, 33.0 g of the product of Example 18 was converted to 43.0 g of N'-(butylaminocarbonyl)-N,N-dimethyl-4-methoxy-1,2-benzenedisulfonamide, obtained as a viscous oil.

NMR (DMSO-$d_6$) δ: 0.6-1.5 (m, 8.7H, 8 butyl hydrogens); 2.6-3.1 (m, 7.6H, 2 butyl hydrogens + $NMe_2$); 3.85 (s, 2.7H, $OCH_3$); 6.7-7.0 (broad t, 0.7H, $CH_2\underline{NH}$); 7.2-8.1 (m, 2.7H, 3 aromatics); ~9.8 (broad, 0.7H, NH).

## Example 20

**N,N-Dimethyl-2-(isocyanatosulfonyl)-4-methoxybenzenesulfonamide**

Using a procedure analogous to that described in Example 10, 19.7 g of the product of Example 19 was converted to 16.0 g of N,N-dimethyl-2-(isocyanatosulfonyl)-4-methoxybenzenesulfonamide, obtained as a viscous oil.

## Example 21

**N-Methyl-N-(1,1,2,2-tetrafluoroethyl)-2-nitrobenzenesulfonamide**

A solution of 72 g of N-methyl-2-nitrobenzenesulfonamide, 35 g of tetrafluoroethylene and 4 g of powdered potassium hydroxide in 150 ml of dimethylformamide was heated at 85° for 3 hours in a pressure vessel. The reaction mixture was cooled to 25° and poured into 800 ml of water. A heavy oil was separated and the aqueous layer extracted with 150 ml of 1-chlorobutane. The heavy oil, the 1-chlorobutane extract and an additional 300 ml of 1-chlorobutane were combined and washed three times with water. The organic solution was dried over magnesium sulfate and concentrated in-vacuo to yield 85.1 g of N-methyl-N-(1,1,2,2-tetrafluoroethyl)-2-nitrobenzenesulfonamide as a viscous orange oil.

NMR$(DCDl_3)\delta$: 3.1-3.3 (m, 2.6H, $N-CH_3$);
5.2-7.3 (tt, 1.0H, JFCH=57Hz and JFCCH=5Hz, $CF_2H$);
7.7-8.3 (m, 4.4H, 4 aromatics).

Example 22

N-Methyl-N-(1,1,2,2-tetrafluoroethyl)-2-aminobenzenesulfonamide

Using a procedure analogous to that described in Example 12, 82.0 g of the product of Example 21 was converted to 65.4 g of N-methyl-N-(1,1,2,2-tetrafluoroethyl)-2-aminobenzenesulfonamide, as a viscous oil.

NMR($CDCl_3$)δ: 2.9 (t, J=3Hz, 2.7H, $CH_3NCF_2-$);
4.5-5.4 (broad, 1.9H, $NH_2$);
5.4-5.6 and 6.4-7.8 (m, 5.4H, 4 aromatics + $CF_2H$).

Example 23

N-Methyl-N-(1,1,2,2-tetrafluoroethyl)-1,2-benzenedisulfonamide

Using a procedure analogous to that described in Example 8, 60.0 g of the product of Example 22 was converted to 32.0 g of N-methyl-N-(1,1,2,2-tetrafluoroethyl)-1,2-benzenedisulfonamide, m.p. 102-107°.

NMR(DMSO-$d_6$)δ: 3.3 (broad singlet, 2.9H, $CH_3N$);
6.05, 6.95, 7.85 (tt, 1.0H, $CF_2H$);
7.5 (broad singlet, 1.8H, $SO_2NH_2$);
7.9-8.7 (m, 4.3H, 4 aromatics).

Anal. Calcd. for $C_9H_{10}N_2O_4S_2F_4$: C, 30.86; H, 2.88; N, 8.00.

Found: C, 31.1; 31.2
H, 2.82, N, 8.01.
2,69 8.04

## Example 24

N'-(Butylaminocarbonyl)-N-methyl-N-(1,1,2,2-tetrafluoroethyl)-1,2-benzenedisulfonamide

Using a procedure analogous to that described in Example 9, 24.5 g of the product of Example 23 was converted to 31.0 g of N'-(butylaminocarbonyl)-N-methyl-N-(1,1,2,2-tetrafluoroethyl)-1,2-benzenedisulfonamide, m.p. 146-152°.

NMR(DMSO-$d_6$)δ: 0.6-1.5 (m, 7.2H, 7 butyl hydrogens);
2.7-3.1 (m, 2.0H, 2 butyl hydrogens);
3.1-3.3 (s, 2.7H, $CH_3N$);
5.9, 6.85, 7.75 (tt, 1.0H, $CF_2H$);
6.65 (t, 1.0H, $CH_2$-NH)
7.9-8.5 (m, 4.3H, 4 aromatics);
10.2 (s, 0.8H, NH).

## Example 25

N-Methyl-N-(1,1,2,2-tetrafluoroethyl)benzenesulfonylisocyanate

Using a procedure analogous to that described in Example 10, the product of Example 24 was converted to N-methyl-N-(1,1,2,2-tetrafluoroethyl)benzenesulfonylisocyanate, isolated as a viscous oil.

IR(Neat): 2230 $cm^{-1}$ ($SO_2NCO$).

## Example 26

N-(Phenylmethoxy)-2-nitrobenzenesulfonamide

A solution of 96.4 g of O-benzylhydroxylamine in 80 ml of tetrahydrofuran was added to a solution of 86.8 g of o-nitrobenzenesulfonyl chloride in 400 ml of tetrahydrofuran at 5-10°. After stirring at ambient temperature for 16 hours the solvent was evaporated. The residue was washed with water, filtered and dried to give 112.6 g of N-(phenylmethoxy)-2-nitrobenzenesulfonamide, m.p. 148-151°.

Example 27

N-Methyl-N-(phenylmethoxy)-2-nitrobenzenesulfonamide

A mixture of 110.6 g of the product of Example 26, 49.8 g of anhydrous potassium carbonate, 55.8 g of methyliodide and 500 ml of acetone was stirred at ambient temperature for 20 hours. The solvent was evaporated and the residue partitioned between methylene chloride and water. Concentration of the organic extract afforded 88.9 g of N-methyl-N-(phenylmethoxy)-2-nitrobenzenesulfonamide as a yellow oil. [NMR: δ 2.8 (s, 3H, $NCH_3$)].

Example 28

N-Methyl-N-(phenylmethoxy)-2-aminobenzenesulfonamide

To a solution of 88.9 g of the product of Example 27 in 565 ml of 90% aqueous acetic acid was added 83 g of iron powder at 80-90° in several portions. Water (60 ml) was then added and heating continued for 20 minutes. Charcoal (10 g) was added and the solution filtered through Celite® and concentrated in-vacuo. The residue was made strongly alkaline with 20% NaOH solution, then extracted with methylene chloride. The extract was dried and concentrated in-vacuo to give 29.3 g of N-methyl-N-(phenylmethoxy)-2-aminobenzenesulfonamide as a yellow oil. [NMR: δ 5.1 (broad singlet, 2H, $NH_2$)].

Example 29

N-Methyl-N-(phenylmethoxy)-1,2-benzenedisulfonamide

Using a procedure analogous to that described in Example 8, 28.8 g of the product of Example 28 was converted to 19.3 g of N-methyl-N-(phenylmethoxy)-1,2-benzenedisulfonamide, m.p. 122-128°. [NMR: δ 5.7 (broad singlet, 2H, $SO_2NH_2$)].

Example 30

N'-(Butylaminocarbonyl]-N-methyl-N-(phenylmethoxy)-1,2-benzenedisulfonamide

Using a procedure analogous to that described in Example 9, 18.8 g of the product of Example 29 was converted to 19.1 g of N-(butylaminocarbonyl)-N-methyl-N-(phenylmethoxy)-1,2-benzenedisulfonamide, obtained as a brown syrup.

This compound could then be converted to the N-methyl-N-(phenyl-methoxy)benzene-2-sulfonylisocyanate.

Using the methods and Examples discussed above, the sulfonylisocyanates and sulfonylisothiocyanates described in Table I can be prepared.

Table I

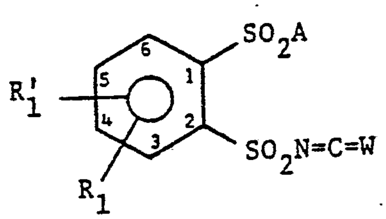

| $R_1'$ | $R_1$ | A | W | $\nu N=C=W$ ($cm^{-1}$) |
|---|---|---|---|---|
| H | H | $-N(CH_3)_2$ | O | 2220 |
| H | H | $-N(C_2H_5)_2$ | O | 2210 |
| H | H | $-N-C_2H_5$<br>$CH_3$ | O | |
| H | H | $-N-\underline{i}-C_3H_7$<br>$CH_3$ | O | |
| H | H | $-N-\underline{n}-C_4H_9$<br>$CH_3$ | O | |
| H | H | $-N-\underline{n}-C_3H_7$<br>$CH_3$ | O | |
| H | H | 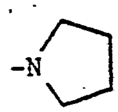 | O | |
| H | H | $-N-CF_2CF_2H$<br>$CH_3$ | O | 2230 |
| H | H | $-N(CF_2CF_2H)_2$ | O | |
| H | 4-Cl | $-N(CH_3)_2$ | O | 2250 |
| H | $4-CF_3$ | $-N(CH_3)_2$ | O | 2220 |
| H | $4-CH_3O-$ | $-N(CH_3)_2$ | O | 2230 |
| H | 4-F- | $-N(CH_3)_2$ | O | 2250 |
| H | $4-CH_3-$ | $-N(CH_3)_2$ | O | 2240 |
| H | $4-NO_2-$ | $-N(CH_3)_2$ | O | |
| H | 4-Br- | $-N(CH_3)_2$ | O | 2280 |
| H | $4-C_2H_5O-$ | $-N(CH_3)_2$ | O | 2250 |

Table I (continued)

| $R_1'$ | $R_1$ | A | W | $\nu N=C=W$ ($cm^{-1}$) |
|---|---|---|---|---|
| H | 4-CN | $-N(CH_3)_2$ | O | |
| H | $4-(CH_3)_2N$ | $-N(CH_3)_2$ | O | |
| H | $4-SCH_3$ | $-N(CH_3)_2$ | O | |
| H | $4-SO_2CH_3$ | $-N(CH_3)_2$ | O | |
| H | H | $-N(CH_3)_2$ | S | |
| $5-CH_3$ | $4-CH_3$ | $-N(CH_3)_2$ | O | |
| $5-CH_3O$ | $4-CH_3O$ | $-N(CH_3)_2$ | O | |
| 5-F | 4-Cl | $-N(CH_3)_2$ | O | |
| 5-Cl | 4-Cl | $-N(CH_3)_2$ | O | |
| H | 3-Cl | $-N(CH_3)_2$ | O | |
| H | 5-Cl | $-N(CH_3)_2$ | O | |
| H | 6-Cl | $-N(CH_3)_2$ | O | |
| H | H | $-NCH_2CN$<br>$CH_3$ | O | |
| H | H | $-N-CH_2CO_2CH_3$<br>$CH_3$ | O | |
| H | H | 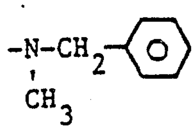 | O | |
| H | H | 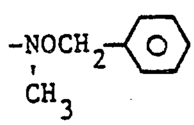 | O | |
| H | H | 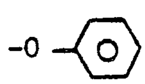 | O | |
| H | H | $-OCH_2CF_3$ | O | |

Table I. (continued)

| $R_1'$ | $R_1$ | A | W | $\nu N=C=W$ ($cm^{-1}$) |
|---|---|---|---|---|
| H | $4\text{-}\overset{O}{\overset{\parallel}{C}}CH_3$ | $-N(CH_3)_2$ | O | |
| H | $4\text{-}\overset{O}{\overset{\parallel}{C}}\text{-}\underline{i}\text{-}C_3H_7$ | $-N(CH_3)_2$ | O | |
| H | $4\text{-}CH_2OCH_3$ | $-N(CH_3)_2$ | O | |
| H | $4\text{-}CH_3O\text{-}\underline{i}\text{-}C_3H_7$ | $-N(CH_3)_2$ | O | |
| H | H | $\underset{CH_3}{N}\text{-}CH_2C\equiv CH$ | O | |
| H | H | $\underset{CH_3}{N}\text{-}C(CH_3)_2C\equiv CH$ | O | |
| H | $4\text{-}CH_2SCH_3$ | $N(CH_3)_2$ | O | |
| H | $4\text{-}CH_2SO_2CH_3$ | $N(CH_3)_2$ | O | |
| H | $4\text{-}CH_2\underset{O}{\underset{\parallel}{S}}CH_3$ | $N(CH_3)_2$ | O | |
| H | H | 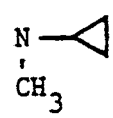 | O | |
| H | H | 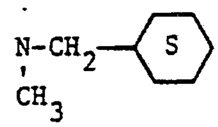 | O | |

Claims:

1. A compound of the general formula

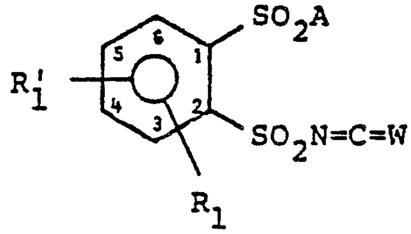

I-B

wherein

A is $NR_2R_3$, $OCH_2CCl_3$, $OCH_2CBr_3$, O 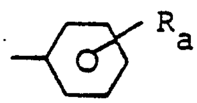

$OCH_2CF_2R_b$ or $\underset{CF_3}{\underset{|}{OCHR_c}}$ where $R_a$ is H, Cl, $CH_3$, $OCH_3$ or $NO_2$ and $R_b$ is H, F or $C_1$-$C_2$ alkyl with 0-5F and $R_c$ is $CH_3$ or $CF_3$;

$R_1$ is H, Cl, Br, F, $C_1$-$C_3$ alkyl, $NO_2$, $OCH_3$, $\overset{O}{\overset{\|}{C}}R_d$, $CH_2OR_d$, $CF_3$, N=C=O, $N(CH_3)_2$, CN, $S(O)_nCH_3$ or $CH_2S(O)_nCH_3$ where n is 0 or 2 and $R_d$ is $C_1$-$C_3$ alkyl;

$R_1'$ is H, Cl, F, Br, $CH_3$ or $OCH_3$;

W is O or S;

$R_2$ is $C_1$-$C_6$ alkyl, $C_3$-$C_4$ alkenyl, $C_3$-$C_6$ cycloalkyl, $C_5$-$C_6$ cycloalkenyl, $C_4$-$C_7$ cycloalkylalkyl, $C_3$-$C_6$ cycloalkyl substituted with 1-2 $CH_3$ groups, $C_3$-$C_5$ alkynyl, $CF_2CF_2H$, $CF_2CHFCl$, $CF_2CHFBr$, $CF_2CHFCH_3$,

$C(CH_3)_2CN$, $(CH_2)_mCN$, where m is 1 or 2, $CH_2CH_2OCH_3$, $CH_2CH(CH_3)OCH_3$, $(CH_2)_3OCH_3$, $CHR_7CO_2R_8$ or $CHR_7CON(R_8)_2$, where $R_7$ is H or $CH_3$ and $R_8$ is $C_1$-$C_3$ alkyl, $OCH_3$,

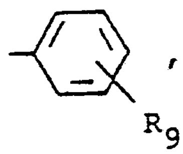 , $CHR_7$ 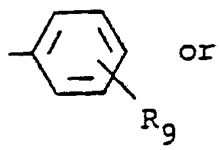 or $OCHR_7$ 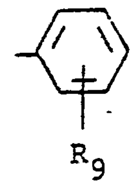

where

$R_9$ is H, $CH_3$, Cl, Br or F;

$R_3$ is $C_1$-$C_4$ alkyl, $C_3$-$C_4$ alkenyl, $CH_2CH_2OCH_3$, $CH_2CH(CH)_3OCH_3$, $CH_2CF_3$ or $(CH_2)_mCN$, where m is 1 or 2 or $CHR_7CO_2R_8$ or $NR_2R_3$ taken together are

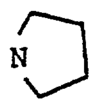 , 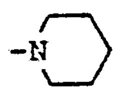 , 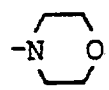 or 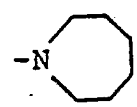

provided that:

1) when $R_2$ is $OCH_3$, $R_3$ is $CH_3$;

2) when $R_2$ is $CF_2CHFCl$, $CF_2CHFBr$, $CF_2CF_2H$ or $CF_2CHFCF_3$, then $R_3$ is $C_1$-$C_4$ alkyl;

3) when $R_1$ is CN or N=C=O, it cannot be in the 3-position.

2. A compound of claim 1 wherein W is O.

3. A compound of claim 2 where $R_1'$ is H.

4. A compound of claim 3 wherein A is $OCH_2CF_3$ or $NR_2R_3$ and $R_2$ is $C_1$-$C_6$ alkyl, $C_3$-$C_4$ alkenyl, $OCH_3$, $CH_2CH_2OCH_3$, $CH_2CH(CH_3)OCH_3$ or $(CH_2)_3OCH_3$, or $NR_2R_3$ taken together are

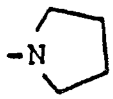 , 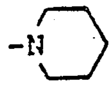 , 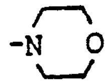 or 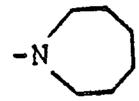

5. A compound of claim 4 wherein $R_3$ is $C_1$-$C_4$ alkyl or wherein $NR_2R_3$ taken together are

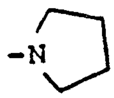 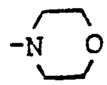 or 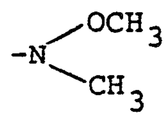

6. A compound of claim 5 wherein $R_1$ is H, Cl, $CF_3$, $NO_2$, $CH_3$ or $OCH_3$.

7. A compound of claim 6 wherein $R_2$ is $C_1$-$C_4$ alkyl or wherein $NR_2R_3$ taken together are

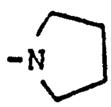 or 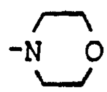

8. A compound of claim 1 which is N,N-dimethyl-2-(isocyanatosulfonyl)benzenesulfonamide.

9. A compound of claim 1 which is N-ethyl-2-(isocyanatosulfonyl)-N-methylbenzenesulfonamide.

10. A compound of claim 1 which is 2-(isocyanatosulfonyl)-N-methyl-N-(methylethyl) benzenesulfonamide.

11. A compound of claim 1 which is N,N-dimethyl-2-(isocyanatosulfonyl)-4-trifluoromethylbenzenesulfonamide.

12. A compound of claim 1 which is 2-[(2,2,2-trifluoroethyl)sulfonyl]benzenesulfonyl isocyanate.

13. A method for the preparation of a compound of claim 1 which comprises reacting a substituted sulfonamide of general formula

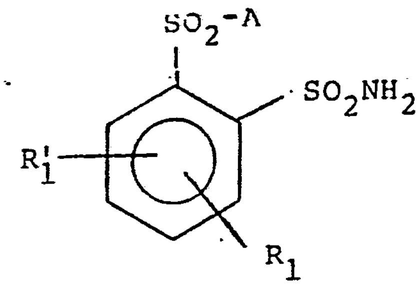

wherein A, $R_1$ and $R_1'$ are as defined in claim 1, with an alkyl isocyanate, phosgene, and a catalytic amount of tertiary amine in an inert solvent:

14. A method for the preparation of a compound of claim 1 which comprises reacting a correspondingly substituted N-alkyl-N' benzenesulfonylurea with phosgene and a catalytic amount of a tertiary amine in an inert solvent.

15. A method for the preparation of a compound of claim 1 which comprises reacting the substituted sulfonamide defined in claim 13 with carbon disulfide and base to form a salt of the corresponding dithiocarbamic acid and reacting said salt with phosgene in the presence of an inert solvent.

16. A compound of claim 1 wherein:

A is $NR_2R_3$;

$R_1$ is H, Cl, Br, F, $NO_2$, $C_1$-$C_3$ alkyl, $OCH_3$, $CF_3$, $N(CH_3)_2$, CN or $S(O)_nCH_3$ where n is O or 2;

$R_1'$ is H;

$R_2$ is as defined in claim 1 other than $C_4$-$C_7$ cycloalkylalkyl, $C_3$-$C_5$ alkynyl, haloalkyl or

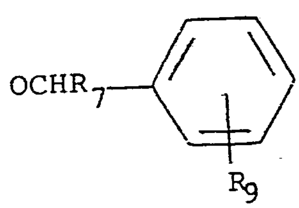

; and

$R_3$ is as defined in claim 1 other than $CH_2CF_3$